(19) Europäisches Patentamt

European Patent Office

Office européen des brevets

(11) **EP 1 340 497 A1**

(12) **EUROPEAN PATENT APPLICATION**

(43) Date of publication:
**03.09.2003 Bulletin 2003/36**

(51) Int Cl.⁷: **A61K 31/337**, A61K 9/107,
A61K 9/48

(21) Application number: **02290513.7**

(22) Date of filing: **01.03.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicants:
• **NOVAGALI SAS**
**91000 Evry (FR)**
• **Yissum Research Development Company of the Hebrew University of Jerusalem**
**91042 Jerusalem (IL)**

(72) Inventors:
• **Lambert, Gregory**
**91370 Verrieres le Buisson (FR)**

• **RazaFindratsita, Alain**
**94550 Chevilly LaRue (FR)**
• **Garrigue, Jean-Sébastian**
**91370 Verrieres le Buisson (FR)**
• **Yang, Shicheng, Dept. of Ind. & Phys. Pharmacy**
**West Lafayette, IN 47907-1336 (US)**
• **Gursoy, Neslihan**
**Jerusalem (IL)**
• **Benita, Simon**
**Mevasseret Zion 90805 (US)**

(74) Representative: **Ahner, Francis et al**
**Cabinet Régimbeau**
**20, rue de Chazelles**
**75847 Paris cedex 17 (FR)**

(54) **Self emulsifying drug delivery systems for poorly soluble drugs**

(57)    A pharmaceutical composition in a form of a self microemulsifying drug delivery system comprising:

- one or more therapeutic agent(s) which have low solubility in water or are water-insoluble,
- vitamin E,
- one co-solvent selected from propylene glycol and ethanol,
- one or more bile salts,
- TPGS, and

- one further surfactant selected from Tyloxapol and polyoxyl hydrogenated castor oil.

EP 1 340 497 A1

**Description**

The present invention relates to a pharmaceutical excipient formulation, more particularly to a pharmaceutical pre-emulsion excipient enhancing the absorption of poorly water soluble drugs, particularly the oral absorption of taxoids.

The clinical use of some drugs is possible only if a drug delivery system is developed to transport them to their therapeutic target in the human body. This problem is particularly critical for water insoluble or poorly water soluble compounds for which direct injections may be impossible.

A few examples of therapeutic substances, which are poorly hydrosoluble, are the following: Palmitoyl Rhizoxin, Penclomedine, Vitamin A and its derivatives (retinoic acid, isotretinoin, etc.), Tamoxifen, Etoposide, Campothecin, Navelbine, Valproic acid, Tacrolimus, Sirolimus (Rapamycin), Cyclosporin A, Clarithromicin, Testosterone, Estradiol, Progesterone, Ciprofloxacine, Fenofibrate, Benzafibrate, Azithromicine, Itraconazole, Miconazole, Propofol, Brimonidine, Latanoprost, and Paclitaxel.

Paclitaxel, one of the best known taxoid, disrupts tubulin dynamics. It has a significant clinical activity against a broad range of tumor types including breast, lung, head and neck, bladder, and platinum-refractory ovarian carcinoma (E. K. Rowinsky. The development and clinical utility of the taxoid class of antimicrotubule chemotherapy agents. *Annu Rev Med.* **48**: 353-74 (1997)). However, paclitaxel has a low therapeutic index. It is a complex diterpenoid product, with a bulky, extended fused ring system as well as a number of hydrophobic substituents, which lead to its poor solubility in water (1 µg/ml) resulting in serious formulation problems (R. T. Liggins, W. L. Hunter, H. M. Burt. Solid-state characterization of paclitaxel. J *Pharm Sci.* **86**: 1458-63 (1997)). It is highly lyophobic and the solubility of paclitaxel in lipophilic solvents, such as soybean oil is quite low and precludes the use of simple oil-in-water emulsions for formulation considerations. The commercially available product, Taxol® , is currently formulated for systemic administration in a mixture of ethanol and polyoxyethylated castor oil; the latter appears to be primarily responsible for drug related hypersensitivity reactions, rather than the drug itself (R. E. Gregory, A. F. De Lisa. Paclitaxel: a new antineoplastic agent for refractory ovarian cancer. *Clin Pharm.* **12**: 401-15 (1993)). Moreover, polyoxyethylated castor oil also causes the nonlinear pharmacokinetic behavior of paclitaxel (A. Sparreboom, O. van Tellingen, W. J. Nooijen, J.H. Beijnen. Nonlinear pharmacokinetics of paclitaxel in mice results from the pharmaceutical vehicle Cremophor EL. Cancer Res. 56: 2112-5 (1996); O. van Tellingen, M. T. Huizing, V. R. Panday, J. H. Schellens, W. J. Nooijen, J. H. Beijnen. Cremophor EL causes (pseudo-) non-linear pharmacokinetics of paclitaxel in patients. *Br J Cancer* **81**: 330-5 (1999)).

The current approaches for reducing the side effects of the actual commercial product are mainly focused on developing formulations that are devoid of polyoxyethylated castor oil. Several attempts have been made to deliver paclitaxel using alternative systems, such as nanoparticles (R. Cavalli, O. Caputo, M. R. Gasco. Preparation and characterization of solid lipid nanospheres containing paclitaxel. *Eur J Pharm Sci.* **10**: 305-9 (2000); S. S. Feng, G. F. Huang, L. Mu. Nanospheres of biodegradable polymers: a system for clinical administration of an anticancer drug paclitaxel (Taxol). [In Process Citation]. *Ann Acad Med Singapore.* **29**: 633-9 (2000)), liposomes (P. Crosasso, M. Ceruti, P. Brusa, S. Arpicco, F. Dosio, L. Cattel. Preparation, characterization and properties of sterically stabilized paclitaxel-containing liposomes. J *Controlled Release.* **63**: 19-30 (2000); A. Sharma, R. M. Straubinger. Novel taxol formulations: preparation and characterization of taxol-containing liposomes. *Pharm Res.* **11**: 889-96 (1994)), water-soluble prodrugs (J. M. Terwogt, B. Nuijen, W. W. T. B. Huinink, J. H. Beijnen. Alternative formulations of paclitaxel. *Cancer Treat Rev.* **23**: 87-95 (1997); A. Pendri, C. D. Conover, R. B. Greenwald. Antitumor activity of paclitaxel-2'-glycinate conjugated to poly(ethylene glycol): a water-soluble prodrug. *Anticancer Drug Des.* **13**: 387-95 (1998)), emulsions (P. P. Constantinides, K. J. Lambert, A. K. Tustian, B. Schneider, S. Lalji, W. Ma, B. Wentzel, D. Kessler, D. Worah, and S. C. Quay. Formulation development and antitumor activity of a filter-sterilizable emulsion of paclitaxel. *Pharm Res.* **17**: 175-82 (2000); B. B. Lundberg. A submicron lipid emulsion coated with amphipathic polyethylene glycol for parenteral administration of paclitaxel (Taxol). *J Pharm Pharmacol.* **49**: 16-21 (1997); P. Kan, Z. B. Chen, C. J. Lee, I. M. Chu. Development of nonionic surfactant/ phospholipid o/w emulsion as a paclitaxel delivery system. J *Controlled Release.* **58**: 271-8 (1999), P. Simamora, R. M. Dannenfelser, S. E. Tabibi, S. H. Yalkowsky. Emulsion formulations for intravenous administration of paclitaxel. *PDA J Pharm Sci Technol.* **52**: 170-2 (1998)) and microspheres (R. T. Liggins, S. D'Amours, J. S. Demetrick, L. S. Machan, H. M. Burt. Paclitaxel loaded poly(L-lactic acid) microspheres for the prevention of intraperitoneal carcinomatosis after a surgical repair and tumor cell spill [In Process Citation]. *Biomaterials.* **21**: 1959-69 (2000); Y. M. Wang, H. Sato, I. Adachi, I. Horikoshi. Preparation and characterization of poly(lactic-co-glycolic acid) microspheres for targeted delivery of a novel anticancer agent, taxol. *Chem Pharm Bull (Tokyo).* **44**: 1935-40 (1996)). However, the success is for the moment still limited. None of these alternatives has reached the stage of replacing polyoxyethylated castor oil based vehicle in the clinical application.

Another approach to overcome the hypersensitivity reactions resulting from polyoxyethylated castor oil can be the design of oral formulations of paclitaxel, even in the presence of polyoxyethylated castor oil, since it is not orally absorbed (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. *Clin Cancer Res.* **5**: 3379-84 (1999)). Oral administration of paclitaxel would, thus, prevent the adverse effects caused by the vehicle substance polyoxyethylated castor oil and offer additional advantages over i.v. administration, including elimination of the need for frequent visits to the outpatient clinic and easier chronic administration (R. T. Dorr. Pharmacology and toxicology of Cremophor EL diluent. *Ann Pharmacother.* **28**: S11-4 (1994)). However, preclinical studies have suggested that paclitaxel is not significantly absorbed after oral administration; the systemic bioavailability in humans after oral paclitaxel administration is less than 6% (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. *Clin Cancer Res.* **5**: 3379-84 (1999)). The explanations proposed to account for the poor oral bioavailability of paclitaxel are multifactorial. The most likely explanations are its affinity for the membrane-bound drug efflux pump P-glycoprotein (P-gp), metabolization by cytochrome P450 and poor water solubility (R. T. Liggins, W. L. Hunter, H. M. Burt. Solid-state characterization of paclitaxel. J *Pharm Sci.* **86**: 1458-63 (1997); J. van Asperen, O. van Tellingen, A. Sparreboom, A. H. Schinkel, P. Borst, W. J. Nooijen, and J. H. Beijnen. Enhanced oral bioavailability of paclitaxel in mice treated with the P-glycoprotein blocker SDZ PSC 833. *Br J Cancer.* **76**: 1181-3 (1997); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. *Clin Cancer Res.* **6**: 3459-68 (2000)). A number of studies have been carried out to verify in both animals and patients if the oral bioavailability of paclitaxel can be greatly improved when the drug is administered with P-gp inhibitors (R. T. Dorr. Pharmacology and toxicology of Cremophor EL diluent. *Ann Pharmacother.* **28**: S11-4 (1994); J. van Asperen, O. van Tellingen, A. Sparreboom, A. H. Schinkel, P. Borst, W. J. Nooijen, and J. H. Beijnen. Enhanced oral bioavailability of paclitaxel in mice treated with the P-glycoprotein blocker SDZ PSC 833. *Br J Cancer.* **76**: 1181-3 (1997); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. *Clin Cancer Res.* **6**: 3459-68 (2000)). Cyclosporine A (CsA), a well-known immunosuppressive agent, was shown to be one of the most promising P-gp inhibitors to enhance the oral absorption of paclitaxel (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. *Clin Cancer Res.* 5: 3379-84 (1999); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. *Clin Cancer Res.* **6**: 3459-68 (2000)). CsA is a registered drug and thus is more readily available for clinical studies. However, the use of CsA for long-term oral dosing may be hindered by its immunosuppressive side effect, which renders this compound less suitable for chronic use in cancer patients most of whom are already immunodeficient because of chemotherapy.

Recently, it was reported that self-emulsifying drug delivery systems (SEDDS) consisting of isotropic mixtures of oil and surfactants could significantly improve the oral availability of poorly absorbed, hydrophobic and/or lipophilic drugs (T. Gershanik, S. Benita. Self-dispersing lipid formulations for improving oral absorption of lipophilic drugs. *Eur J Pharm Biopharm.* **50**: 179-88 (2000)). SEDDS are composed of natural or synthetic oils, surfactants and one or more hydrophilic solvents and co-solvents. The principal characteristic of SEDDS is their ability to form fine oil-in-water emulsions or microemulsions upon mild agitation following dilution by aqueous phases. These formulations can disperse in the gastrointestinal lumen to form microemulsions or fine emulsions, upon dilution with gastrointestinal fluids. In *in-vivo* absorption studies in non-fasting dogs, SEDDS elicited at least a three-fold greater $C_{max}$ and AUC of a lipophilic naphthalene derivative than that of the drug in any other dosage form (N. H. Shah, M. T. Carvajal, C. I. Patel, M. H. Infeld, A. W. Malick. Self-emulsifying drug delivery systems (SEDDS) with polyglycolyzed glycerides for improving *in vitro* dissolution and oral absorption of lipophilic drugs. *Int J Pharm.* **106**: 15-23 (1994)). The absorption of ontazolast in rats was significantly enhanced by all lipid-based formulations (D. J. Hauss, S. E. Fogal, J. V. Ficorilli, C. A. Price, T. Roy, A. A. Jayaraj, and J. J. Kierns. Lipid-based delivery systems for improving the bioavailability and lymphatic transport of a poorly water-soluble LTB4 inhibitor. *J Pharm Sci.* **87**: 164-9 (1998)). Microemulsions have successfully been used to improve drug solubilization/dissolution and/or intestinal absorption of poorly absorbed drugs including CsA pr(P. P. Constantinides. Lipid microemulsions for improving drug dissolution and oral absorption: physical and biopharmaceutical aspects. *Pharm Res.* **12**: 1561-72 (1995); S. Tenjarla. Microemulsions: an overview and pharmaceutical applications. *Crit Rev Ther Drug Carrier Syst.* **16**: 461-521 (1999)).

The objective of the present invention is to provide a pharmaceutical composition in the form of a self micro-emulsifying drug delivery system comprising bile salts, for example sodium deoxycholate.

The invention is directed to a pharmaceutical composition comprising:

- one or more therapeutic agent(s) which have low solubility in water or are water-insoluble,
- vitamin E,
- one co-solvent selected from propylene glycol and ethanol,
- one or more bile salts, for example sodium deoxycholate,
- TPGS, and
- one further surfactant selected from Tyloxapol and polyoxyl hydrogenated castor oil.

In an advantageous embodiment, the bile salt is sodium deoxycholate.

In another advantageous embodiment, the one or more bile salts represent 1 to 40% (w/w) of the final composition. For example, the sodium deoxycholate represents 1 to 40% (w/w) of the final composition;

In another advantageous embodiment, the vitamin E is from 2 to 6% (w/w) of the final composition.

According to the present invention, therapeutic agents are any compound which has a biological activity and is soluble in the oil phase. Said therapeutic agents may be antibiotics, analgesics, antidepressants, antipsychotics, hormones or chemotherapeutics.

Agents like taxoids, i.e. paclitaxel, docetaxel, their derivatives, analogs and prodrugs are preferred.

Preferred compositions according to the invention contain a relative proportion of paclitaxel between 0.5 and 4% (w/w), preferably equal to 3% (w/w).

Preferred pharmaceutical composition according to the instant invention comprises an emulsion including vitamin E, D-α-tocopheryl polyethylene glycol succinate 1000 (TPGS), polyoxyl hydrogenated castor oil and at least, one therapeutic agent, the relative proportions of vitamin E, TPGS and polyoxyl hydrogenated castor oil being respectively 10-60, 40-90, 10-80 (w/w) of the total oil phase, more preferably 10-45, 10-65 and 10-60.

Another preferred pharmaceutical composition according to the invention contains a relative proportion of vitamin E, TPGS, sodium deoxycholate and Tyloxapol are respectively 2-6, 5-60, 1-40 and 5-70 (w/w) of the total oil phase.

More preferably, the relative proportions of vitamin E, TPGS, sodium deoxycholate and Tyloxapol are respectively 3-5, 20-35, 2-20 and 20-40% (w/w) of the total oil phase.

Concerning co-solvent, the relative proportions of propylene glycol are in the range of 0-50% (w/w) of the final formulation, preferably equal to 20% (w/w) and the relative proportions of ethanol are in the range of 5-50% (w/w) of the final formulation, preferably equal to 30% (w/w).

The composition according to the invention may be associated with any pharmaceutical excipient to form a dosage form, which can be administered to animals or humans via intravascular, oral, intramuscular, cutaneous and subcutaneous routes. Specifically emulsions according to the invention can be given by any of the following routes among others: intra-abdominal, intra-arterial, intra-articular, intra-capsular, intra-cervical, intra-cranial, intra-ductal, intra-dural, intra-lesional, intra-ocular, intra-locular, intra-lumbar, intra-mural, intra-operative, intra-parietal, intra-peritoneal, intra-plural, intra-pulmonary, intra-spinal, intra-thoracic, intra-tracheal, intra-tympanic, intra-uterine, intra-ventricular or transdermal or can be nebulised using suitable aerosol propellants.

The microemulsifying compositions according to the instant invention may be used for the treatment of different diseases like cancers, tumours, Kaposi's sarcoma, malignancies, uncontrolled tissue or cellular proliferation secondary to tissue injury, and any other disease conditions responsive to taxoids such as paclitaxel and docetaxel, and/or prodrugs and derivatives of the foregoing. Among the types of carcinoma which may be treated particularly effectively with oral paclitaxel, docetaxel, other taxoids, and their prodrugs and derivatives, are hepatocellular carcinoma and liver metastases, cancers of the gastrointestinal tract, pancreas, prostate and lung, and Kaposi's sarcoma. Examples of non-cancerous disease conditions which may be effectively treated with these active agents administered orally in accordance with the present invention are uncontrolled tissue or cellular proliferation secondary to tissue injury, polycystic kidney disease, inflammatory diseases (e. g., arthritis) and malaria.

The novel compositions may be administered in any known pharmaceutical oral dosage form. For example, the formulations may be encapsulated in a soft or hard gelatin capsule or may be administered in the form of a liquid oily preparation. Each dosage form may include, apart from the essential components of the composition conventional pharmaceutical excipients, diluents, sweeteners, flavouring agents, colouring agents and any other inert ingredients regularly included in dosage forms intended for oral administration (see e. g., Remington's Pharmaceutical Sciences, 17th Ed., 1985).

Precise amounts of each of the target drugs included in the oral dosage forms will vary depending on the age, weight, disease and condition of the patient.

Although some of the oral formulations of the invention may provide therapeutic blood levels of the taxoid active ingredient when administered alone, an advantageous method of the invention for treating mammalian patients (particularly human patients) suffering from taxoid-responsive disease conditions is to administer the oral formulations containing the taxoid target agent concomitantly with the administration of at least one dose of an oral bioavailability enhancing agent. An other advantageous method of the invention for treating mammalian patients is to administer the oral formulations containing the taxoid target agent concomitantly or separately with another antitumor agent like carboplatinum and the like.

The preferred embodiment of the method of the invention for oral administration to humans of paclitaxel, its derivatives, analogs and prodrugs, and other taxoids comprises the oral administration of an oral absorption or bioavailability enhancing agent to a human patient simultaneously with, or prior to, or both simultaneously with and prior to the oral administration to increase the quantity of absorption of the intact target agent into the bloodstream.

Different advantages of the present invention will be readily appreciated with the following tables, drawings and examples.

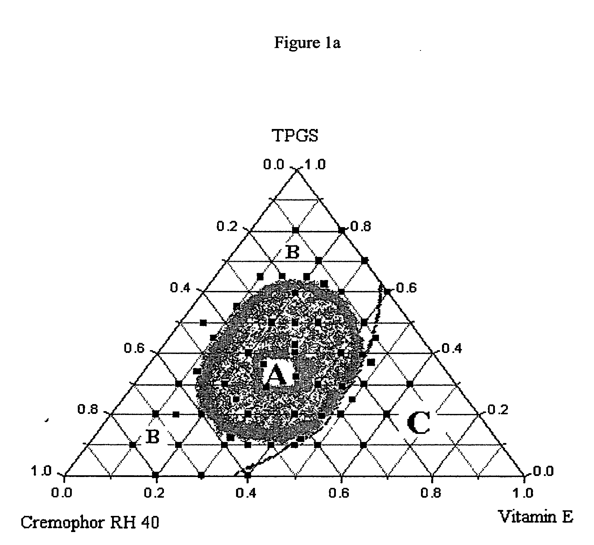

**Figure 1a** illustrates the pseudo-ternary diagram of SEDDS containing 1.25% paclitaxel, 10% DOC-Na, and 20% propylene glycol following 1:10 dilution with water.

A: microemulsions and/or micellar solutions stable for at least 6 hours with no paclitaxel precipitation.

B: microemulsions and/or micellar solutions, but, paclitaxel precipitation within 6 hours.

C: emulsions or opaque dispersions with droplet size larger than 100 nm, whereas, no paclitaxel precipitation noted within 6 hours.

**Figure 1b** illustrates the pseudo-ternary diagram of SEDDS containing 5% vitamin E, 30% ethanol and 3% paclitaxel following 1:10 dilution with water.

A: microemulsions and/or micellar solutions stable for at least 2 hours with no paclitaxel precipitation, with droplet size in the range 1 to 10 nm.

B: submicron emulsions stable for at least 2 hours with no paclitaxel precipitation, with droplet size in the range of 40 to 400 nm.

**Figure 2** illustrates the drug logarithmic concentration-time profiles after intravenous administration of Taxol® and paclitaxel SEDDS: (a) 2, (b) 5 and (c) 10 mg/kg paclitaxel. Data are expressed as means ± S.D. (n = 3).

**Figure 3** illustrates the plasma paclitaxel concentration-time profiles after oral administration at the doses of (A) 2, (B) 5 and (C) 10 mg/kg paclitaxel, and (D) 2 mg/kg paclitaxel of SEDDS with 40 mg/kg CsA. Data are expressed as means ± S.D. (n = 3).

**Figure 4** illustrates the relationship between dose-adjusted AUC and administered dose for: (A) intravenous and (B) oral administration.

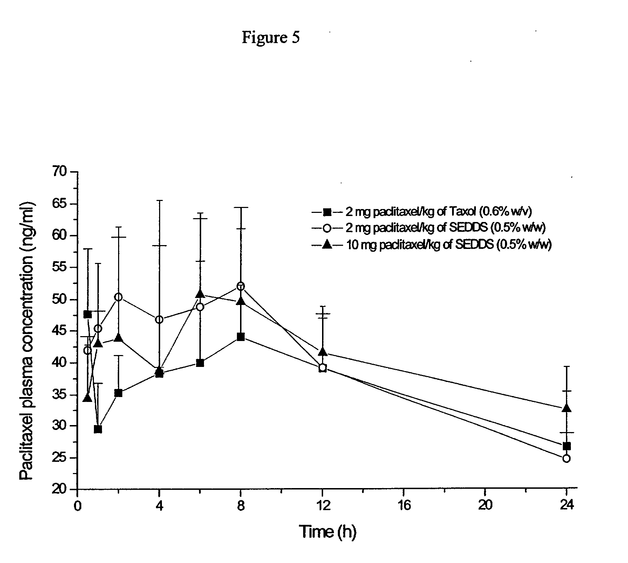

**Figure 5** illustrates the plasma paclitaxel concentration-time profiles after oral administration at constant drug concentration in the SEDDS administered at different doses.

**EXAMPLE 1: CHARACTERIZATION OF PACLITAXEL EMULSIONS FOLLOWING 1:10 DILUTION WITH WATER**

**1. MATERIALS AND METHODS**

**1.1 Materials**

Paclitaxel (MW 853) with 99.34% (w/w) purity (HPLC) was purchased from Farmachem (Lugano, Switzerland). Vitamin E, deoxycholic acid sodium salt (DOC-Na) and Tyloxapol were bought from Sigma (St. Louis, MO, USA). D-$\alpha$-tocopheryl polyethylene glycol succinate 1000 (TPGS) was a gift from Eastman Chemical (Kingsport, TN, USA). Ethanol was bought from SDS (Peypin, France). All solvents were HPLC grade.

**1.2 Methods**

**1.2.1. Preparation of Paclitaxel SEDDS with polyoxyl hydrogenated castor oil**

The blank formulation consisted of vitamin E, TPGS, polyoxyl hydrogenated castor oil, DOC-Na and propylene glycol. Paclitaxel was weighed and added to the blank formulation and thoroughly mixed to form a clear homogenous mixture. Paclitaxel emulsions may be formed following 1:10 dilution of SEDDS with distilled water. A ternary phase diagram study (S. Watnasirichaikule, N. M. Davies, T. Rades, I. G. Tucker. Preparation of biodegradable insulin nanocapsules from biocompatible microemulsions. *Pharm* Res. **17**: 684-9 (2000); M. Trotta, E. Ugazio, M. R. Gasco. Pseudo-ternary phase diagrams of lecithin-based microemulsions: influence of monoalkylphosphates. J *Pharm Pharmacol.* **47**: 451-4 (1995)) was carried out to identify the optimal SEDDS containing paclitaxel at different concentrations ranging from 0.5 to 2.5% (w/w). In the triangular phase diagram study, vitamin E, TPGS and polyoxyl hydrogenated castor oil concentrations were varied, while, the DOC-Na and propylene glycol concentrations remained constant at 10 and 20% (w/w), respectively, in all compositions. The values shown in the diagram for each excipient were always calculated in percentage from the total combination which, in fact, represented only 70% of the final composition.

**1.2.2. Preparation of paclitaxel SEDDS with Tyloxapol**

The vitamin E and TPGS are mixed and DOC-Na is added in one hand. In the other hand, Tyloxapol and ethanol are mixed and paclitaxel is dissolved in said mixture. The drug containing Tyloxapol and ethanol mixture is added to vitamin E-TPGS-DOC-Na mixture to form a clear homogeneous oily mixture. Paclitaxel emulsion may be formed by dilution of SEDDS with distilled water.

Ternary phase diagram study was carried out as disclosed before. In the pseudo-ternary phase diagram study, DOC-Na, TPGS and Tyloxapol concentration were varied while the ethanol, vitamin E and paclitaxel concentrations remain constant in all compositions. The values shown in the diagram for each excipient were always calculated in percentages from the combination of the three excipients. This combination represents 62% of the final composition while ethanol, vitamin E and paclitaxel represent 38%.

- Droplet size

Emulsions were formed following 1:10 dilution of paclitaxel SEDDS with distilled water. The droplet size of the resultant emulsions was determined by the PCS method using a Coulter® Model $N_4$SD (FL, USA).

- Zeta Potential

The Zeta potential of the resultant emulsions after 1:10 dilution of SEDDS with water was measured by a Malvern Zetasizer 3000 (Malvern, UK).

- Stability Study

Optimal SEDDS formulations obtained in 1.2.1. containing 0.5, 1.0, 1.5, 2.0, 2.5 and 3% (w/w) paclitaxel were prepared. Microemulsions were formed after 1:10 dilution with pre-warmed distilled water (37 °C) and then stored at 37 °C to monitor the possible precipitation of paclitaxel. The chemical stability of paclitaxel in SEDDS containing 0.5 - 3% w/w paclitaxel at 4 and 25 °C was monitored using an analytical HPLC method (M. Andreeva, P. D. Iedmann, L. Binder, V. W. Armstrong, H. Meden, M. Binder, M. Oellerich. A simple and reliable reversed-phase high-performance liquid chromatographic procedure for determination of paclitaxel (taxol) in human serum. *Ther Drug Monit.* **19**: 327-32 (1997); A. Sharma, W. D. Conway, R. M. Straubinger. Reversed-phase high-performance liquid chromatographic determination of taxol in mouse plasma. *J Chromatogr B Biomed Appl. 655:* **315**-9 (1994)).

## 2. RESULTS

### 2.1 SEDDS Preparation

#### 2.1.a SEDDS with polyoxyl hydrogenated castor oil

Fig. 1a shows that the formulations containing 1.25% paclitaxel, 10% DOC-Na and 20% propylene glycol in shaded area A of the ternary diagram can form microemulsions and/or micellar solutions following 1:10 dilution with water. The resultant microemulsions or micellar solutions can remain physically stable for at least 6 hours with no paclitaxel precipitation. The combination of vitamin E (28.5% w/w), TPGS (43.0% w/w) and polyoxyl hydrogenated castor oil (28.5% w/w), located at the center of area A, was chosen as the optimal formulation. The corresponding optimal blank formulation, therefore, consisted of (%, w/w) vitamin E (20), TPGS (30), polyoxyl hydrogenated castor oil (20), DOC-Na (10) and propylene glycol (20).

The formulations located in area B following aqueous dilution (1:10) do form microemulsions or micellar solutions, but paclitaxel precipitation appeared within 6 hours.

The formulations located at area C can form emulsions or opaque dispersions with main droplet size larger than 100 nm whereas no paclitaxel precipitate was noted within 6 hours.

Vitamin E used in paclitaxel SEDDS forms the oil phase in the resultant microemulsions after dilution of SEDDS with an aqueous phase. Vitamin E may not only improve the incorporation of paclitaxel into SEDDS to form stable microemulsions, but also might produce beneficial protective effects by quenching free radicals (K. Kline, W. Yu, B. G. Sanders. Vitamin E: mechanisms of action as tumor cell growth inhibitors. *J Nutr.* **131**: 161S-163S (2001)). Furthermore, TPGS, a water-soluble surfactant, inhibits the P-gp efflux system, and thus, have a beneficial effect in improving the oral absorption of paclitaxel (R. J. Sokol, et al. Improvement of cyclosporin absorption in children after liver transplantation by means of water-soluble vitamin E. *Lancet.* **338**: 212-4 (1991) and J. M. Dintaman, J. A. Silverman. Inhibition of P-glycoprotein by D-alpha-tocopheryl polyethylene glycol 1000 succinate (TPGS). *Pharm Res.* **16**: 1550-6 (1999)).

#### 2.1.b. SEEDS with Tyloxapol

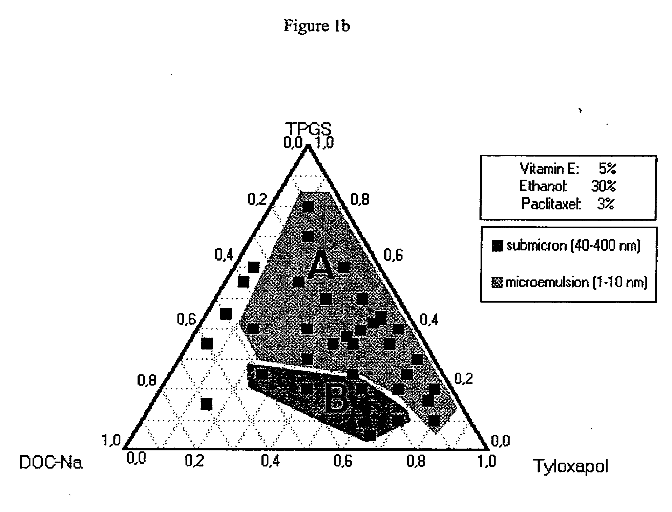

Fig. 1b shows that the formulations containing 3% paclitaxel, 5% vitamin E and 30% ethanol in grey area A of the ternary diagram can form microemulsions and/or micellar solutions following 1:10 dilution with water. The resultant microemulsions or micellar solutions, with droplet size in the range of 1 to 10 nm, can remain physically stable for at least 2 hours with no paclitaxel precipitation. The combinations of TPGS (20-35% w/w), sodium deoxycholate (2-20% w/w) and Tyloxapol (20-40% w/w), located near the center of area A, were chosen as the optimal formulations.

The formulations located in dark grey area B of the ternary diagram following aqueous dilution (1:10) do form submicron emulsions with droplet size in the range of 40 to 400 nm, with no paclitaxel precipitation for at least 2 hours.

### 2.2 Physicochemical Characterization

#### 2.2.a. SEDDS with polyoxyl hydrogenated castor oil

Following 1:10 dilution of paclitaxel SEDDS (1.25% w/w) in distilled water, the droplet size of the resultant microemulsions was 31.5 ± 4.0 nm for unimodal results and 1.0 ± 0.4 nm for SDP weight results. The resultant microemulsions were negatively charged, and the zeta potential value was - 45.5 ± 0.5 mV.

### 2.2.b. SEEDS with Tyloxapol

Following 1:10 dilution of paclitaxel SEDDS in distilled water (3% w/w), the droplet size of the resultant microemulsions was 0.95 ± 0.09 nm.

### 2.3 Stability Study

Following 1:10 dilution of optimal SEDDS containing various concentrations of paclitaxel, the precipitation of the drug from the resultant microemulsions depended on the initial concentration of paclitaxel in SEDDS. The physical stability of paclitaxel in microemulsions decreased with the increase of paclitaxel concentration in SEDDS formulations. When paclitaxel concentrations were lower than 1.25% w/w, the precipitation time was longer than 6 h. No precipitate was noted when the concentration was 0.5% w/w over 6 months. When paclitaxel concentration was higher than 1.5% w/w, the drug precipitated easily from the resultant microemulsions. The precipitation time was about 2 h as the concentration was elevated to 2.5% w/w.

The preliminary chemical stability studies indicated that paclitaxel in the negatively charged SEDDS was stable at 4 and 25 °C. The drug content in SEDDS at 4 and 25 °C did not change over three months.

### EXAMPLE 2: PHARMACOKINETICS OF PACLITAXEL SEDDS

### 1. MATERIALS AND METHODS

### 1.1 Animal Study

Experiments were performed on male Sprague-Dawley (S.D.) rats weighing 200-250 g, which fasted overnight for 12-14 hours with free access to water. Experimental procedures were approved by the Hebrew University of Jerusalem Committee on Use and Care of Animals. Following 1:10 dilution of SEDDS containing 0.5, 1.25 or 2.5% paclitaxel with water (oral) or saline (intravenous), the microemulsions were orally or intravenously administered at doses of 2.0, 5.0 or 10.0 mg/kg paclitaxel, respectively. In studies where indicated, CsA (40 mg/kg, Neoral® ; Novartis, Basel, Switzerland) was orally administered 10 min before oral administration of paclitaxel SEDDS. Blood samples were collected into heparinized tubes at time points of 0.5, 1, 2, 4, 6, 8, 12 and 24 hours after oral dosing. Additional samples were collected at 1, 5, 15 min, 1.5 h and 3 h post intravenous dosing. At each time point, three rats were sacrificed to take the blood. All blood samples were immediately placed on ice upon collection and centrifuged at 4000 rpm for 15 min to obtain the plasma. Aliquots were stored at - 20°C until analysis.

### 1.2 Analysis of Paclitaxel

Prior to extraction, 0.05-2.0 ml of rat plasma that was diluted to a total of 2.0 ml with double distilled water for intravenous administration or 2.0 ml plasma for oral administration was mixed with 0.3 μg Taxotere® , dissolved in 50 μl methanol, used as an internal standard. Extraction of paclitaxel was accomplished by adding 4.0 ml of *tert*-butyl methyl ether and vortex-mixing the sample for 1.0 min. The mixture was then centrifuged for 10 min at 4000 rpm, after which 3.0 ml of the organic layer was transferred to a clean tube and evaporated to dryness under vacuum using a Labconco Vortex Evaporator (Lumitron Electronic Instrument Ltd., MO, USA) at 20 °C. Approximately 200 μl mobile phase was used to reconstitute the residue and 80 μl aliquot was injected into the HPLC equipped with a Hypersil® BDS $C_{18}$ (5μm, 250 × 4.6 mm; Alltech, Deerfield, IL, USA) analytical column and a Betasil $C_{18}$ (5μm, 10 × 4.6 mm; Alltech, Deerfield, IL, USA) guard column. The detection wavelength of paclitaxel was 227 nm (M. Andreeva, P. D. Iedmann, L. Binder, V. W. Armstrong, H. Meden, M. Binder, M. Oellerich. A simple and reliable reverse-phase high-performance liquid chromatographic procedure for determination of paclitaxel (taxol) in human serum. *Ther Drug Monit.* **19**: 327-32 (1997); A. Sharma, W. D. Conway, R. M. Straubinger. Reversed-phase high-performance liquid chromatographic determination of taxol in mouse plasma. *J Chromatogr B Biomed Appl.* **655**: 315-9 (1994)). The mobile phase was acetonitrile-water (48:52) and pumped at the flowrate of 1.5 ml/min. The analysis was carried out at room temperature. The retention time of paclitaxel and docetaxel was 12.4 and 11.0 min, respectively. The lower limit of quantification for paclitaxel was 10 ng/ml, and the range of linear response was 50-800 ng/ml ($r^2$ >0.9990). The observed recovery of paclitaxel was 96.8-101.6%, and the intra-day and inter-day assay variabilities were less than 5.6%.

### 1.3 Pharmacokinetic Data Analysis

Pharmacokinetic parameters in plasma were obtained from the pooled concentration-time data of each experiment with statistical moment algorithm using the WinNonlin program package. The $AUC_{0-24}$ from time 0 to time 24 h ($T_{24}$) was calculated using the linear trapezoidal method, and $AUC_{0-\infty}$ was calculated by dividing the concentration of 24 h data point ($C_{24}$) by the elimination rate constant (k) as follows: $AUC_{0-\infty}$ =$AUC_{0-24}$ + $C_{24}$/k.

The area under the first moment curve (AUMC) was calculated as follows:

$$AUCM_{0-\infty} = AUMC_{0-24} + (T_{24}\cdot C_{24})/k + C_{24}/k$$

The relative bioavailability (Fr) and systemic (absolute) bioavailability (Fa) were calculated as follows:

$$Fr = [(AUC_{SEDDS})_{oral}/(AUC_{Taxol})_{oral}]_{\,0-\infty}$$

$$Fa = [(AUC_{test})_{oral}/(AUC_{Taxol})_{i.v.}]_{\,0-\infty}$$

Thus, fr and fa,, the relative and absolute bioavailability at 24 h, respectively, were calculated as follows:

$$fr = [(AUC_{SEDDS})_{oral}/(AUC_{Taxol})_{oral}]_{0-24}$$

$$fa = [(AUC_{test})_{oral}/(AUC_{Taxol})_{i.v.}]_{0-24}.$$

The mean residence time (MRT) was determined by dividing $AUMC_{0-\infty}$ by $AUC_{0-\infty}$.

## 2. RESULTS

### 2.1 Intravenous Administration

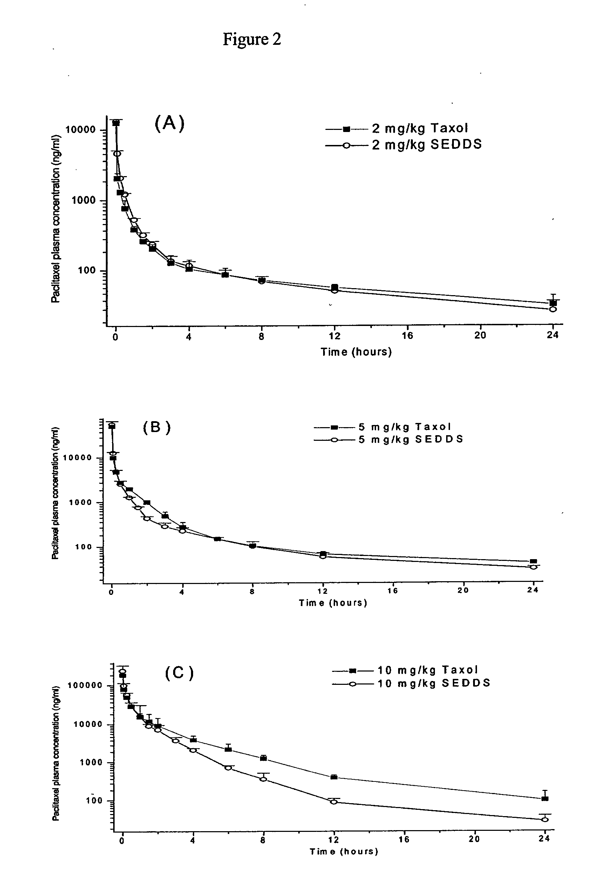

Paclitaxel plasma concentration data obtained following the i.v. administration were analyzed by the non-compartment and two-compartment models. Figs. 2 (A), (B) and (C) show the drug logarithmic concentration-time profiles after i.v. administration of Taxol® and paclitaxel SEDDS at the doses of 2, 5 and 10 mg/kg, respectively. The relevant pharmacokinetic parameters are outlined in Table 1.

The clearance (Cl) of paclitaxel in Taxol® was 513.6, 433.8 and 118.3 (ml/h·kg) at the doses of 2, 5 and 10 mg/kg, respectively. The clearance of paclitaxel in SEDDS was 455.4, 493.6 and 137.3 (ml/h·kg) at the doses of 2, 5 and 10 mg/kg, respectively. The $AUC_{0-\infty}$ of paclitaxel in Taxol® was 3894.4 (ng·h/ml) at the dose of 2 mg/kg, and it increased to 11525.5 (ng·h/ml) at the dose of 5 mg/kg and 84517.5 (ng·h/ml) at the dose of 10 mg/kg. The $AUC_{0-\infty}$ of paclitaxel in SEDDS was 4392.1(ng.h/ml) at the dose of 2 mg/kg, and it escalated to 10129.9 (ng·h/ml) at the dose of 5 mg/kg and 72846.3 (ng·h/ml) at the dose of 10 mg/kg. The maximum concentration ($C_{max}$) and $AUC_{0-\infty}$ of paclitaxel increased disproportionately with higher doses, and the clearance of paclitaxel decreased with the increase in dose, indicating a nonlinear or saturable pharmacokinetic behavior for Taxol® and SEDDS. The MRT and steady-state volume of distribution ($V_{ss}$) decreased with the increase of the dose, but the MRT and $V_{ss}$ of paclitaxel SEDDS were lower compared to Taxol® .

The absolute bioavailability of paclitaxel SEDDS was 112.1% at the dose of 2 mg/kg, and it decreased to 87.9% at the dose of 5 mg/kg and to 86.2% at the dose of 10 mg/kg.

Taxol® showed serious toxicity problems in the present study, and about 30% of the rats died at the dose of 10 mg/kg. On the contrary, there were no side effects of paclitaxel SEDDS at the same dose.

### 2.2 Oral Administration

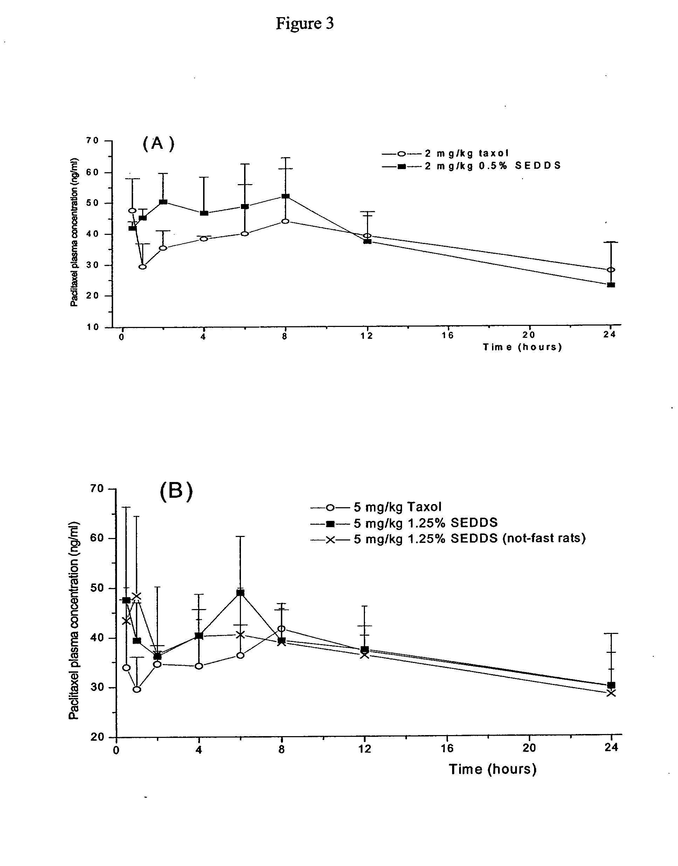

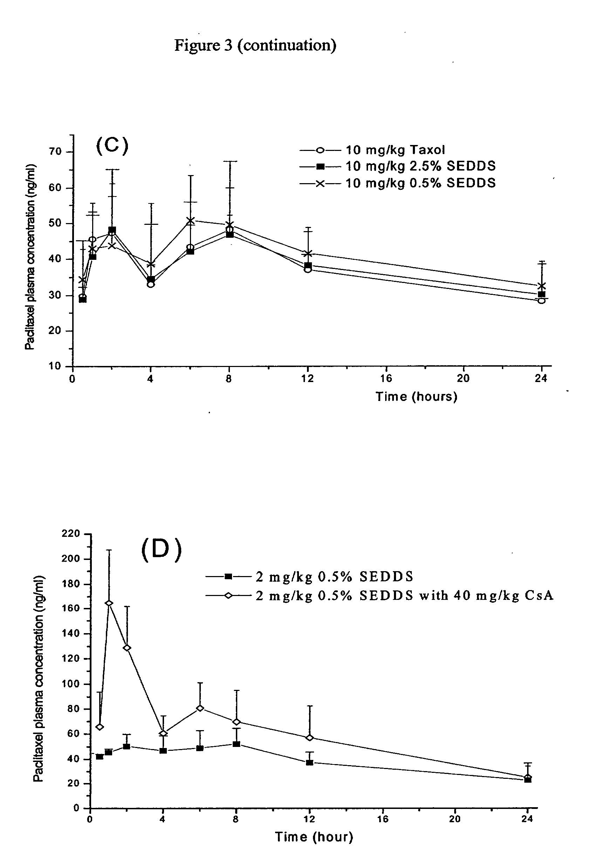

Figs. 3 (A), (B), and (C) show the plasma paclitaxel concentration-time profiles after oral administration at the doses of 2, 5 and 10 mg/kg paclitaxel, respectively.

Paclitaxel plasma concentration-time profiles after oral administration of 2 mg/kg paclitaxel SEDDS with 40 mg/kg CsA is shown in Fig 3 (D). Table 2 shows the relevant pharmacokinetic parameters calculated using non compartmental analysis.

Following 1:10 dilution of paclitaxel SEDDS with distilled water, the resultant microemulsions were orally administered to rats immediately. The values of $C_{max}$ were between 40 and 60 ng/ml, except for the paclitaxel SEDDS co-administered with CsA (160 ng/ml).

Compared with Taxol® , the $AUC_{0-24}$ of paclitaxel SEDDS increased slightly at all indicated doses. The fr of paclitaxel SEDDS between 0 and 24 h increased ranging from 1.5 to 10%, and this increase was inversely proportional to the increase in dose (Table 2). The fa of paclitaxel SEDDS between 0 and 24 h was as high as 28.1% at the dose of 2 mg/kg, and then decreased to 8.3% at the dose of 5 mg/kg and 1.1% at the dose of 10 mg/kg. However, taken Taxol® as the standard formulation for the evaluation of bioavailability of paclitaxel SEDDS, the relative bioavailability (Fr) of paclitaxel SEDDS increased by 1.5% at the dose of 2 mg/kg, but it increased by 43.8% and 14.4% at the doses of 5 and 10 mg/kg, respectively. The absolute bioavailability (Fa) was 42.7, 22.2 and 1.0% at the doses of 2, 5 and 10 mg/kg paclitaxel, respectively.

Compared to the fasted rats, the AUC of paclitaxel SEDDS in non-fasted rats at the dose of 5 mg/kg showed a little decrease, but it was higher than that of Taxol® . This result indicated that there was a slight influence of food intake on the absorption of paclitaxel. For the same dose (10 mg/kg paclitaxel) but different concentrations (0.5 and 2.5% w/w) of paclitaxel in SEDDS, the AUC of SEDDS with 0.5 % w/w paclitaxel was higher than that of SEDDS with 2.5% w/w paclitaxel (Fig. 3C). This indicated that the excipient concentration could slightly improve the absorption of paclitaxel in SEDDS. When co-administered with CsA (40 mg/kg), the $AUC_{0-24}$ of paclitaxel SEDDS increased 1.73 fold compared with that of Taxol® and 1.59 fold compared with that of SEDDS without CsA at the dose of 2 mg/kg paclitaxel. Moreover, the $C_{max}$ significantly increased and reached the therapeutic level (0.1 μmol, equivalent

to 85 ng/ml). The duration of plasma concentration above 0.1 μmol lasted nearly 4.0 h after oral paclitaxel SEDDS administration at the dose of 2 mg/kg co-administered with 40 mg/kg CsA, but this threshold was not reached after oral administration of Taxol® or SEDDS alone following a single dose administration. The relative bioavailability (Fr) of paclitaxel SEDDS with CsA was 133.9% and the absolute bioavailability (Fa) was 56.4% at the dose of 2 mg/kg. This indicates that CsA can greatly increase the bioavailability of paclitaxel confirming previous reported results (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. *Clin Cancer Res.* **5**: 3379-84 (1999); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. *Clin Cancer Res.* **6**: 3459-68 (2000)).

The $MRT_{0-24}$ of paclitaxel SEDDS was similar to that of Taxol® at all indicated doses, except that of paclitaxel SEDDS co-administered with CsA. The $MRT_{0-\infty}$ of paclitaxel SEDDS increased at high doses compared with that of Taxol® . The $MRT_{0-24}$ and $MRT_{0-\infty}$ of paclitaxel SEDDS co-administered with CsA were the shortest among all oral formulations at various doses.

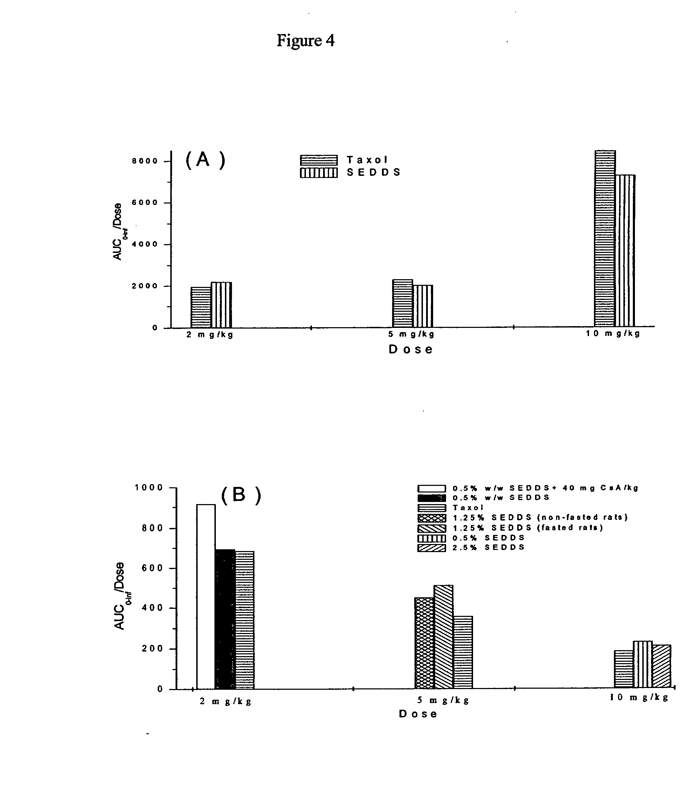

As compared with Taxol®, the relative bioavailability of paclitaxel SEDDS increased by 43.8% at the dose of 5 mg/kg, and by 14.1 and 25.1% at the dose of 10 mg/kg for the SEDDS formulation containing 2.5 and 0.5% w/w paclitaxel, respectively. For the same concentration of excipients in the formulation, the dose of 5 mg/kg paclitaxel has achieved the highest bioavailability (Table 2), indicating that 1.25% w/w paclitaxel in SEDDS was the optimal concentration. The disproportionate decrease of mean $C_{max}$ and $AUC_{0-\infty}$ (Figure 4B) values with the increase of the dose suggested that there is a saturable process in the absorption of oral paclitaxel.

This study shows that paclitaxel microemulsions can form following 1:10 dilution of SEDDS with an aqueous phase. The resulting microemulsions bear a negative or a positive charge. The oral bioavailability of paclitaxel could be improved significantly when paclitaxel was formulated into SEDDS.

An additional advantage of the SEDDS can be found in the convenience and compliance of the patient. In order to administer an oral dose of 90 mg/m² paclitaxel twice daily, 30 ml of the commercially available Taxol® would be required. This might bring with itself the possibility of precipitation of the drug and make the commercial viability of the product more difficult. However, such a problem would not be a concern with the SEDDS used in the present study since the administered dose of about 180 mg, required for a 90 mg/m² dose, would be only 6-12 ml dissolved in a glass of water, provided the concentration of the drug used is 2.5-1.25% w/w, respectively. And finally, the improved Fr and Fa values indicate that SEDDS is a promising system for improving the oral bioavailability of paclitaxel.

**Claims**

1. A pharmaceutical composition in a form of a self microemulsifying drug delivery system comprising:

   - one or more therapeutic agent(s) which have low solubility in water or are water-insoluble,
   - vitamin E,
   - one co-solvent selected from propylene glycol and ethanol,
   - one or more bile salts,
   - TPGS, and
   - one further surfactant selected from Tyloxapol and polyoxyl hydrogenated castor oil.

2. A pharmaceutical composition according to claim 1, wherein the bile salt is sodium deoxycholate.

3. A pharmaceutical composition according to claim 2, wherein the sodium deoxycholate represents 1 to 40% (w/w) of the final composition.

4. A pharmaceutical composition according to anyone of claims 1 to 3, wherein vitamin E is from 2 to 6% (w/w) of the final composition.

5. A pharmaceutical composition according to anyone of claims 1 to 4, wherein the therapeutic agent is a chemotherapeutic agent.

6. A pharmaceutical composition according to claim 5, wherein the chemotherapeutic agent is a taxoid.

7. A pharmaceutical composition according to claim 6, wherein the taxoid is selected from paclitaxel, docetaxel, their derivatives, analogs and prodrugs.

8. A pharmaceutical composition according to claim 7, wherein the taxoid is paclitaxel.

9. A pharmaceutical composition according to claim 8, wherein the relative proportion of paclitaxel is between 0.5 and 4% (w/w).

10. A pharmaceutical composition according to claim 9, wherein the relative proportion of paclitaxel is 3% (w/w).

11. A pharmaceutical composition according to anyone of claims 1 to 10 comprising at least one therapeutic agent, wherein the relative proportions of vitamin E, TPGS and polyoxyl hydrogenated castor oil are respectively 10-60, 40-90 and 10-80 (w/w) of the total oil phase.

**12.** A pharmaceutical composition according to claim 11 wherein the relative proportions of vitamin E, TPGS and polyoxyl hydrogenated castor oil are respectively 10-45, 10-65 and 10-60 (w/w) of the total oil phase.

**13.** A pharmaceutical composition according to anyone of claims 1 to 10, wherein the relative proportions of vitamin E, TPGS, sodium deoxycholate and Tyloxapol are respectively 2-6, 5-60, 1-40 and 5-70 (w/w) of the total oil phase.

**14.** A pharmaceutical composition according to claim 13, wherein the relative proportions of vitamin E, TPGS, sodium deoxycholate and Tyloxapol are respectively 3-5, 20-35, 2-20 and 20-40 (w/w) of the total oil phase.

**15.** A pharmaceutical composition according to claims 1 to 14, wherein the relative proportions of propylene glycol are in the range of 0-50% (w/w) of the final formulation, preferably equal to 20% (w/w) and the relative proportions of ethanol are in the range of 5-50% (w/w) of the final formulation, preferably equal to 30% (w/w).

**16.** A pharmaceutical dosage form comprising a self emulsifying composition according to anyone of claims 1 to 15 and pharmaceutical excipients.

**17.** A pharmaceutical dosage form according to claim 16, which is suitable for the oral route.

**18.** An oral pharmaceutical dosage form according to claim 17, wherein the composition is encapsulated in a soft or in a hard gelatin capsule.

**19.** An oral pharmaceutical dosage form according to claim 18, wherein the composition is a liquid oily preparation.

**20.** Use of a self-microemulsifying composition according to anyone of claims 1 to 19 for the manufacture of a medicament useful in the treatment of taxoid-responsive diseases.

**21.** Use accordingly to claim 20 for administration to patients receiving simultaneously with, or prior to, bioavailability enhancing agent and/or another anti-tumor agent.

Figure 1a

Figure 1b

Figure 2

Figure 3

Figure 3 (continuation)

Figure 4

Figure 5

European Patent
Office

**PARTIAL EUROPEAN SEARCH REPORT**

which under Rule 45 of the European Patent Convention
shall be considered, for the purposes of subsequent
proceedings, as the European search report

Application Number

EP 02 29 0513

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 99 45918 A (NAPRO BIOTHERAPEUTICS INC) 16 September 1999 (1999-09-16) * page 2, last line – page 4, line 7; examples 1,2,7,11 * | 1-21 | A61K31/337 A61K9/107 A61K9/48 |
| A | WO 98 30205 A (SONUS PHARMA INC) 16 July 1998 (1998-07-16) * page 5, line 25 – page 6, last line * * page 11, line 6 – line 11 * * page 16, line 7 – page 17, line 18 * * page 18, line 1 – line 17; examples 1,3,4,6-10,22,23,29,30 * | 1-21 | |
| A | WO 99 04787 A (DAVIS STANLEY STEWART ;DANBIOSYST UK (GB); HAN JIHONG (GB)) 4 February 1999 (1999-02-04) * page 1, line 8 – line 19 * * page 2, line 6 – line 17 * * page 3, line 10 – line 19 * * page 6, line 26 – page 7, line 19 * * page 8, line 21 – page 9, line 3; claims 1-7; examples 1,2 * | 1-21 | |

TECHNICAL FIELDS
SEARCHED      (Int.Cl.7)

A61K

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 1 August 2002 | Marttin, E |

| European Patent Office | INCOMPLETE SEARCH SHEET C | Application Number EP 02 29 0513 |
|---|---|---|

```
Claim(s) searched completely:
      1-6,8-21

Claim(s) searched incompletely:
      7

Reason for the limitation of the search:

Present claim 7 relates to an extremely large number of possible
compounds, namely derivatives, analogs and prodrugs of paclitaxel or
docetaxel. Support within the meaning of Article 84 EPC and/or disclosure
within the meaning of Article 83 EPC is to be found, however, for only a
very small proportion of the compounds claimed. In the present case, the
claims so lack support, and the application so lacks disclosure, that a
meaningful search over the whole of the claimed scope is impossible.
Consequently, the search has been carried out for those parts of the
claim  which appears to be supported and disclosed, namely those parts
relating to the compounds paclitaxel and docetaxel as mentioned in claim
7 and the examples.
```

European Patent
Office

**PARTIAL EUROPEAN SEARCH REPORT**

Application Number

EP 02 29 0513

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | US 5 496 811 A (AVIV HAIM  ET AL) 5 March 1996 (1996-03-05) * column 4, line 12 - line 34 * * column 5, line 62 - column 6, line 26; examples 1,6-8 *<br>--- | 1-12 | |
| A | WO 99 06024 A (MOROZOWICH WALTER ;UPJOHN CO (US); GAO PING (US)) 11 February 1999 (1999-02-11) * page 3, line 8 - line 20 * * page 8, line 9 - line 11; claims 1,2,14-16,19-22,30; examples 5,33-37,44 *<br>----- | 1-12 | |
| | | | TECHNICAL FIELDS SEARCHED (Int.Cl.7) |

EPO FORM 1503 03.82 (P04C10)

## ANNEX TO THE EUROPEAN SEARCH REPORT
## ON EUROPEAN PATENT APPLICATION NO.

EP 02 29 0513

This annex lists the patent family members relating to the patent documents cited in the above–mentioned European search report.
The members are as contained in the European Patent Office EDP file on
The European Patent Office is in no way liable for these particulars which are merely given for the purpose of information.

01-08-2002

| Patent document cited in search report | | | Publication date | Patent family member(s) | | Publication date |
|---|---|---|---|---|---|---|
| WO 9945918 | A | | 16-09-1999 | AU | 2902299 A | 27-09-1999 |
| | | | | BR | 9904856 A | 18-07-2000 |
| | | | | CN | 1255852 T | 07-06-2000 |
| | | | | EP | 0977562 A1 | 09-02-2000 |
| | | | | JP | 2001524988 T | 04-12-2001 |
| | | | | WO | 9945918 A1 | 16-09-1999 |
| | | | | US | 2001029264 A1 | 11-10-2001 |
| | | | | ZA | 9901885 A | 10-09-1999 |
| WO 9830205 | A | | 16-07-1998 | EP | 0981328 A1 | 01-03-2000 |
| | | | | JP | 2001508445 T | 26-06-2001 |
| | | | | WO | 9830205 A1 | 16-07-1998 |
| | | | | AU | 5731498 A | 03-08-1998 |
| | | | | ZA | 9800098 A | 08-07-1998 |
| WO 9904787 | A | | 04-02-1999 | AU | 8456198 A | 16-02-1999 |
| | | | | EP | 0999833 A1 | 17-05-2000 |
| | | | | WO | 9904787 A1 | 04-02-1999 |
| | | | | ZA | 9806645 A | 24-01-2000 |
| US 5496811 | A | | 05-03-1996 | AT | 191641 T | 15-04-2000 |
| | | | | AU | 680813 B2 | 14-08-1997 |
| | | | | AU | 3432593 A | 29-03-1994 |
| | | | | CA | 2142103 A1 | 17-03-1994 |
| | | | | DE | 69328368 D1 | 18-05-2000 |
| | | | | DE | 69328368 T2 | 10-08-2000 |
| | | | | EP | 0656779 A1 | 14-06-1995 |
| | | | | SG | 49746 A1 | 15-06-1998 |
| | | | | WO | 9405298 A1 | 17-03-1994 |
| | | | | ZA | 9300069 A | 04-08-1993 |
| | | | | JP | 8508975 T | 24-09-1996 |
| WO 9906024 | A | | 11-02-1999 | AU | 728626 B2 | 11-01-2001 |
| | | | | AU | 8573898 A | 22-02-1999 |
| | | | | AU | 728698 B2 | 18-01-2001 |
| | | | | AU | 8573998 A | 22-02-1999 |
| | | | | BR | 9810729 A | 08-08-2000 |
| | | | | BR | 9811058 A | 05-09-2000 |
| | | | | CN | 1261790 T | 02-08-2000 |
| | | | | CN | 1261796 T | 02-08-2000 |
| | | | | EP | 0989851 A1 | 05-04-2000 |
| | | | | EP | 0999826 A1 | 17-05-2000 |
| | | | | FI | 20000170 A | 28-01-2000 |
| | | | | FI | 20000172 A | 28-01-2000 |
| | | | | HU | 0002440 A2 | 28-09-2001 |
| | | | | JP | 2002510330 T | 02-04-2002 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 02 29 0513

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2002

| Patent document cited in search report | Publication date | Patent family member(s) | Publication date |
|---|---|---|---|
| WO 9906024          A | | JP  2002511099 T | 09-04-2002 |
| | | NO   20000466 A | 28-03-2000 |
| | | NO   20000467 A | 29-03-2000 |
| | | NZ     502566 A | 28-03-2002 |
| | | NZ     502569 A | 31-05-2002 |
| | | PL     338335 A1 | 23-10-2000 |
| | | PL     338509 A1 | 06-11-2000 |
| | | SK     162000 A3 | 11-12-2000 |
| | | SK     172000 A3 | 11-07-2000 |
| | | WO   9906044 A1 | 11-02-1999 |
| | | WO   9906024 A1 | 11-02-1999 |
| | | US   6231887 B1 | 15-05-2001 |
| | | AT     215370 T | 15-04-2002 |
| | | AU     728695 B2 | 18-01-2001 |
| | | AU    8573798 A | 22-02-1999 |
| | | BR    9810866 A | 26-09-2000 |
| | | CN    1261797 T | 02-08-2000 |
| | | DE   69804624 D1 | 08-05-2002 |
| | | DK     999838 T3 | 08-07-2002 |
| | | EP    0999838 A1 | 17-05-2000 |
| | | FI    20000171 A | 28-01-2000 |
| | | HU    0002486 A2 | 28-12-2000 |
| | | JP  2002510329 T | 02-04-2002 |
| | | NO   20000465 A | 28-03-2000 |
| | | NZ     502567 A | 28-03-2002 |
| | | PL     338334 A1 | 23-10-2000 |
| | | SK     182000 A3 | 11-12-2000 |
| | | WO   9906043 A1 | 11-02-1999 |
| | | US   6121313 A | 19-09-2000 |